(19) **Europäisches Patentamt / European Patent Office / Office européen des brevets**

(11) **EP 4 164 617 B1**

(12) **EUROPEAN PATENT SPECIFICATION**

(45) Date of publication and mention of the grant of the patent:
**27.09.2023 Bulletin 2023/39**

(21) Application number: **21732033.2**

(22) Date of filing: **11.06.2021**

(51) International Patent Classification (IPC):
*A61K 9/51* (2006.01)     *A61C 5/50* (2017.01)
*A61K 6/54* (2020.01)     *A61K 6/849* (2020.01)
*A61K 33/08* (2006.01)    *A61P 1/02* (2006.01)
*A61P 31/04* (2006.01)

(52) Cooperative Patent Classification (CPC):
**A61K 9/5153; A61C 5/50; A61K 6/56; A61K 6/849; A61K 9/5192; A61K 33/08; A61P 1/02; A61P 31/04;** A61K 9/19

(86) International application number:
**PCT/EP2021/065768**

(87) International publication number:
**WO 2021/250236 (16.12.2021 Gazette 2021/50)**

(54) **COMPOSITION COMPRISING NANOPARTICLES, METHOD FOR THE PREPARATION OF A COMPOSITION COMPRISING NANOPARTICLES AND USES OF THE COMPOSITION FOR DENTAL TREATMENT**

ZUSAMMENSETZUNG MIT NANOPARTIKELN, VERFAHREN ZUR HERSTELLUNG EINER ZUSAMMENSETZUNG MIT NANOPARTIKELN UND VERWENDUNGEN DER ZUSAMMENSETZUNG ZUR ZAHNÄRZTLICHEN BEHANDLUNG

COMPOSITION COMPRENANT DES NANOPARTICULES, PROCÉDÉ POUR LA PRÉPARATION D'UNE COMPOSITION COMPRENANT DES NANOPARTICULES ET UTILISATION DE LA COMPOSITION POUR LE TRAITEMENT DENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2020 EP 20382504**

(43) Date of publication of application:
**19.04.2023 Bulletin 2023/16**

(73) Proprietors:
  • **Universitat Internacional De Catalunya, Fundació Privada**
    **08017 Barcelona (ES)**
  • **Universitat de Barcelona**
    **08028 Barcelona (ES)**

(72) Inventors:
  • **DURÁN-SINDREU TEROL, Fernando Salvador**
    **08024 Barcelona (ES)**
  • **GONZÁLEZ SÁNCHEZ, José Antonio**
    **08173 Sant Cugat del Vallès (ES)**
  • **PÉREZ ANTOÑANZAS, Román**
    **08850 Gavà (ES)**
  • **ELMSMARI, Firas**
    **08022 Barcelona (ES)**
  • **SÁNCHEZ LÓPEZ, Elena**
    **08028 Barcelona (ES)**
  • **GARCÍA LÓPEZ, María Luisa**
    **08028 Barcelona (ES)**

(74) Representative: **Torner, Juncosa I Associats, SL**
    **C / Pau Claris, 108, 1r 1a**
    **08009 Barcelona (ES)**

(56) References cited:
  • **CERDA-CRISTERNA B I ET AL: "PLGA-based nanoparticles for sustained release of Ca++ for apexification", DENTAL MATERIALS, vol. 32, 12 October 2016 (2016-10-12), - 16 December 2016 (2016-12-16), pages e38-e39, XP029768191, ISSN: 0109-5641, DOI: 10.1016/J.DENTAL.2016.08.079 cited in the application**

**(Cont. next page)**

EP 4 164 617 B1

- **CERDA-CRISTERNA BERNARDINO ISAAC ET AL: "Sustained release of calcium hydroxide from poly(dl-lactide-co-glycolide) acid microspheres for apexification", ODONTOLOGY, SPRINGER JAPAN, TOKYO, vol. 104, no. 3, 15 July 2015 (2015-07-15) , pages 318-323, XP036053597, ISSN: 1618-1247, DOI: 10.1007/S10266-015-0213-6 [retrieved on 2015-07-15]**

## Description

Field of the Invention

The present invention relates to a composition, in particular for antibacterial dental treatment, comprising polymeric nanoparticles. The invention also relates to a method for the preparation of such composition and uses of the composition for dental treatment, in particular for an endodontic treatment.

Background of the Invention

The use of nanoparticles is a constantly expanding field and plays a very important role in the medical field. In recent years, nanoparticles are being commonly used in drug administration (drug delivery applications), since these systems allow to direct a wide variety of molecules to different tissues releasing them sustainably over time.

In dental applications, successful prognosis in endodontics is about 85 % when a pulp infection exists; therefore, it is necessary to establish mechanisms for increasing the success rate. One of the means to achieve it is by using antibacterial intra-canal therapies, among which is the use of calcium hydroxide. However, calcium hydroxide products used up to now present limitations such as a buffer effect or a non-controlled release, quite possibly due to the structural composition thereof.

Some patents and/or patent applications are known which use nanoparticles for dental treatment. For example, from patent US 8105086 a biomaterial for a dental and medical treatment and its use for sealing and/or filling the tooth and bone are known. The calcium salt, calcium oxide, calcium silicate and calcium phosphate compounds are mixed with a water-based solution, and a mixture enriched with bioactive phosphate and calcium is prepared. The mixture, in the form of nanoparticles, comprises a high concentration of water-soluble calcium and phosphate and, accordingly, forms hydroxyapatite during and after the setting. The biomaterial is biocompatible, antibacterial and is capable of forming an effective seal against the re-entry of microorganisms in the cavity.

Likewise, microparticles for a medical treatment are also known in the state of the art. For example, in patent application US 2017135960, a solid microparticle (for example, non-porous) is disclosed, for ocular therapy in particular, loaded with a drug. Processes are also described for producing the superficially-treated microparticle and injectable formulations which include the superficially-treated microparticle.

Likewise, patent KR 101879399 refers to a method for producing polymeric microparticles using dialkyl carbonate (a non-halogenated organic solvent for solubilizing a polymeric compound). The microparticles can efficiently enclose drugs at various concentrations.

Moreover, in the document "PLGA-based nanoparticles for sustained release of Ca++ for apexification", B.I. Cerda-Cristerna et al. Abstracts of the Academy of Dental Materials Annual Meeting, 12-15 October 2016 - Chicago, USA, nanoparticles of Poly(L(+)-lactic acid-co-glycolic) or PLGA for the sustained release of Ca++ for apexification are presented. These PLGA nanoparticles, unlike the present invention, do not present a percentage of efficiency of association of the active compound to the nanoparticles; therefore the authors do not present any confirmation that the nanoparticles include the active principle (CaOH) encapsulated. Furthermore, as indicated in the study, the nanoparticles used have an initial neutral pH of 7.51 and an acid pH at the end of the experiment of 2.44. Therefore, due to this acidification of the nanoparticles over time, they cannot be used in dental disinfection treatments.

Document Cerda-Cristerna et al. "Sustained release of calcium hydroxide from poly(DL-lactide-co-glycolide) acid microspheres for apexification", Odontology 2016, Sep. 104(3):318-23 discloses a study formulating and characterizing calcium hydroxide-PLGA microspheres. Said document describes a method form making PLGA particles encapsulating Ca(OH)$_2$ using an O/W technique and emulsion solvent evaporation technique. Document Xiaoman et al. "Chitosan-decorated calcium hydroxide microcapsules with pH-triggered release for endodontic applications", J Mater Chem B. 2015 Dec 7;3(45):8884-8891 discloses calcium hydroxide microcapsules coated with chitosan and ethylcellulose. Drug release from the microcapsules is pH-triggered.

Therefore, there are no known nanoparticles of polymeric material, particularly Poly(L(+)lactic acid) or PLGA, which include encapsulated calcium hydroxide as an antimicrobial agent and that maintain a high pH over time.

Hence, new compositions are required for dental treatment that includes such nanoparticles and new methods for preparing the nanoparticles.

Disclosure of the Invention

To that end, exemplary embodiments of the present invention provide, according to a first aspect, a composition which comprises nanoparticles. The nanoparticles are formed by a polymeric material and include an antimicrobial agent, particularly calcium hydroxide. According to the present invention, the polymeric material is selected from the group comprising PLGA, polylactic acid (PLA), polyglycolic acid (PGA), polycaprolactone (PCL) and/or combinations thereof.

The calcium hydroxide is encapsulated in the nanoparticles and is in the range from 0.1 to 5 mg/ml. Furthermore, the composition has a pH in the range of 8 - 13. This pH is beneficial because calcium hydroxide has antimicrobial activity due to its basic pH.

By using the referred nanoparticles, the present invention allows to control the dosage, the release and the target penetration of the calcium hydroxide.

In some embodiments, the nanoparticles can include calcium hydroxide on its surface.

In an embodiment, the polymeric material is PLGA. The PLGA can be comprised in a range from 5 to 20 mg/ml in said composition. Preferably, the PLGA will be comprised between 10 and 12 mg/ml.

In particular, the composition/formulation is in an aqueous solution. However, in an embodiment, it can be lyophilized, so that it may be resuspended at the time of use, thereby increasing its stability.

The nanoparticles can have a size between 150 and 300 nanometers. In particular, the size of the nanoparticle is about 150 nanometers. This way, irritation phenomena, collapse of filters or other materials are avoided and the correct encapsulation and the sustained release of the calcium hydroxide are guaranteed.

In an embodiment, the nanoparticles have a polydispersity index lower than 0.2 and a negative surface charge.

According to the present invention, the polymeric material can be biodegradable. Likewise, the composition can be in the form of a gel, an aqueous solution or it can be in the form of dispersed particles.

Embodiments of the present invention also provide, according to a second aspect, a method for preparing a composition comprising nanoparticles. The method comprises:

- preparing an organic phase comprising adding a first mixture, including calcium hydroxide and a first solvent, to a second mixture, including a polymeric material and a second solvent material, wherein the polymeric material can include PLGA, PLA, PGA, PCL, or even combinations thereof;

- preparing an aqueous phase comprising a surfactant product and leaving the aqueous phase under magnetic stirring at a certain stirring speed;

- adjusting a pH of the aqueous phase to a value above 10;

- adding the organic phase into the aqueous phase, drop by drop at a constant rate during a first predetermined time, wherein the mixture is left under magnetic stirring during a second predetermined time;

- carrying out a low-pressure evaporation process for removing said first and second solvent materials; and

- obtaining the nanoparticles.

In an embodiment, the method also comprises lyophilizing the obtained nanoparticles. That is, extracting the water from the nanoparticles by a sublimation process, thus increasing the stability of the nanoparticles.

In an embodiment, the first solvent comprises dimethyl sulfoxide (DMSO) and the second solvent comprises acetone. In an exemplary embodiment, the method comprises using 1 ml of DMSO and 4 ml of acetone.

In an embodiment, the calcium hydroxide is comprised in a range from 0.1 to 5 mg/ml in the first mixture and the polymeric material, PLGA in particular, is comprised in a range from 5 to 20 mg/ml in the second mixture. Likewise, the surfactant is comprised in a range from 5 to 15 mg/ml.

The constant drop rate of the organic phase into the aqueous phase can vary between 2 and 5 seconds, preferably 3 seconds. Likewise, each drop has a volume of 50 microliters.

In an embodiment, the pH value of the aqueous phase is adjusted to a value about 11-12.

In a particular exemplary embodiment, the calcium hydroxide is present in a concentration of 1.7 mg/ml, the polymeric material (PLGA) in 11.5 mg/ml and the surfactant (Poloxamer 188) in 11 mg/ml dissolved in a aqueous buffer prepared at pH 12. By means of this process, a nanoparticle average size of 199.9 $\pm$ 1.35 nm is obtained. Likewise, the nanoparticles, in this particular exemplary embodiment, have a polydispersity index of 0.174 $\pm$ 0.021; a zeta potential of -6.41 $\pm$ 0.10 mV and an association efficiency of 24.98 %.

In a third aspect of the invention, there is provided a use of the composition according to the invention for the following dental treatments: as an antibacterial compound for its placement inside the root canal, as a sealant of the root canal, for pulp capping, for repairing the dentin or dental-periodontal communication, as a material for the retrograde obturation in an apical surgery, as indirect pulp capping and as cavitary base.

Brief Description of the Drawings

The foregoing and other characteristics and advantages will be more fully understood from the following detailed description of some exemplary embodiments, merely illustrative and non-limiting, with reference to the attached drawings, wherein:

Fig. 1 schematically depicts a method for preparing the nanoparticles, PLGA in this case, encapsulating calcium hydroxide as an active molecule or antimicrobial agent, according to an embodiment of the present invention.

Fig. 2 schematically depicts the process of filtration-centrifugation for quantifying non-encapsulated calcium hydroxide.

Detailed Description of the Invention and of Exemplary Embodiments

The present invention provides a composition, particularly for antibacterial dental treatment, comprising nanoparticles of biodegradable polymeric material, for example PLGA, PLA, PGA, PCL, or combinations thereof. In particular, the nanoparticles are of PLGA.

The nanoparticles are of nanospherical type with encapsulated calcium hydroxide. The composition comprising the nanoparticles has a pH value in the range of 8-13, preferably 9-13, more preferably 10-13.

Particularly, the nanoparticles are also enclosed by a surfactant material and comprise a certain quantity of non-encapsulated calcium hydroxide which is located around the surface of each nanoparticle. Subsequent to the method for preparing the nanoparticles, an additional quantity of $Ca(OH)_2$ is added as well as 5% of hydroxypropyl-$\beta$-cyclodextrin. Optionally, 15% of mannitol may also be added.

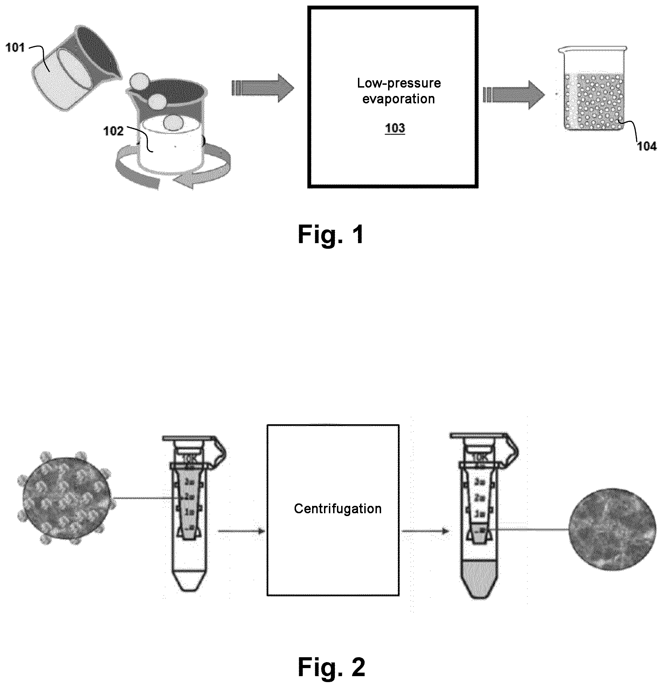

With reference to Fig. 1, therein an embodiment of the displacement method of the solvent used for elaborating the nanoparticles, PLGA in this case, is illustrated. To that end, in a first phase or organic phase 101, the method comprises separately preparing:

- calcium hydroxide in 1 ml of anhydrous DMSO; and
- poly(lactic-co-glycolic acid)or PLGA in 4 ml of acetone.

Subsequently, DMSO containing the calcium hydroxide is added to the 4 ml of acetone.

Thereafter, the second phase or aqueous phase 102 is prepared. According to this embodiment, this phase contains Poloxamer 188 (BASF Chemicals) as surfactant in a concentration of about 11 mg/ml. This aqueous phase is made of a buffer solution adjusted to pH 12. The aqueous phase is left under magnetic stirring adding a magnetic core at 400 r.p.m. per minute.

Next, the organic phase 101 is added to the aqueous phase 102, which is under magnetic stirring. The addition is carried out dropwise at a constant rate, adding, for example, a drop in the center of the stirring cone every 3 seconds. The total time this process takes is approximately 5 minutes.

Then, the mixture is left under magnetic stirring for another 10 minutes. Afterwards, it is evaporated at a reduced pressure 103 (Buchi rotavapor), 9 kPa, for 20 minutes, approximately, to remove the organic solvents used. 10 ml of the formulation of dispersed nanoparticles in water 104 are obtained.

In an embodiment, to achieve greater stability, the obtained nanoparticles are lyophilized. Lyophilizing consists in the extraction of water from the sample by means of a sublimation process. To that end, substances (cryoprotectants) which protect the nanoparticles against the aggression of the process are added and it consists of three steps: freezing, primary drying (here, the water of the nanoparticle is sublimated by using the low pressures), secondary drying (temperature is increased up to 20 degrees Celsius, leaving the pressure of the previous step constant, thereby also evaporating the structural water of the nanoparticles). Advantageously in the present invention it has been proved that after the lyophilization the nanoparticles maintain their initial physical-chemical characteristics. It has been found that by re-suspending the nanoparticles in a liquid medium after lyophilization, the original pH value is obtained, i.e. between 8 and 13.

The key parameters in the composition/formulation have been studied by creating a design space. To that end, the optimization has been carried out by means of a central factorial design created by the Statgraphics Centurion programme. Therein, the concentration parameters of the indicated compounds are optimized, as well as the pH of the aqueous phase 102. The influence in the average size, the polydispersity index, the Zeta potential (carried out by means of laser-Doppler electrophoresis with the M3 PALS system) and the calcium hydroxide association efficiency to the nanoparticles are studied. Thus, the tendencies the nanoparticles follow, as well as the corresponding response superficies, are obtained, which is necessary for achieving a definitive formulation guaranteeing that it is the best formulation that can be obtained.

The optimized nanoparticles present an average size lower than 300 nm, a polydispersity index lower than 0.2, characteristic of the unimodal systems, and a negative surface charge (approximately of-20.0 mV). The polydispersity index is analyzed with a zetasizer model Zetasizer Nano ZS by means of the dynamic light scattering technique. Additionally, the purpose is to encapsulate the maximum quantity possible of calcium hydroxide within the nanoparticles. The encapsulation efficiency (EE) of the nanoparticles is measured indirectly by means of the separation of the encapsulation-free drug by means of filtration-centrifugation (Fig. 2). The fraction of free calcium hydroxide is quantified and, using the following equation, the encapsulated quantity is derived:

$$EE\ (\%) = \frac{Initial\ calcium\ hydroxide\ concentration - Free\ calcium\ hydroxide\ concentration}{Initial\ calcium\ hydroxide\ concentration} \cdot 100$$

For the characterization of the nanoparticles, the drug-polymer interactions have been studied using various analysis techniques such as infrared spectroscopy (FTIR), differential scanning calorimetry (DSC) and X-ray diffraction of the freeze-dried samples. Additionally, the liquid samples have been visualized by means of transmission electronic microscopy (TEM) after a negative staining.

Likewise, in some embodiments, it has been proven by confocal microscopy that the nanoparticles penetrate more than the free drug.

EXAMPLE: pH stability

To test pH stability of the composition according to the present invention, nanoparticles comprising calcium hydroxide and PLGA were studied. An aqueous buffer at pH 12 was prepared, by preparing 100 mL of 0,05 M $Na_2HPO_4$, 53,8 mL of 0,1 M NaOH and adding water up to 200 mL. Poloxamer 188 was solved in 10 mL of the previously mentioned buffer obtaining a concentration of 11 mg/mL. The organic phase was prepared by mixing Calcium hydroxide with 1 mL of DMSO and PLGA with 4 ml of acetone. Afterwards, both were mixed obtaining an organic phase. This organic phase was added dropwise on the water phase under magnetic stirring. Afterwards, the organic solvents were evaporated under reduced pressure. The final concentration of calcium hydroxide was 1,7 mg/mL and 11,5 mg/mL of PLGA.

The aqueous buffer had an initial pH of 12, and pH of the composition according to the invention was measured using a pH meter for a total of 18 days, yielding values over 7.5 during the entire measurement scope. The following table shows the values of the composition from 0 to 432 hours.

| Time (hours) | pH |
|---|---|
| 0 | 9,65 |
| 0,5 | 9,49 |
| 1 | 9,45 |
| 2 | 9,38 |
| 3 | 9,38 |
| 5 | 9,20 |
| 24 | 9,12 |
| 27 | 9,05 |
| 48 | 9,72 |
| 72 | 9,80 |
| 168 | 8,72 |
| 192 | 8,38 |
| 216 | 8,25 |
| 240 | 8,17 |
| 288 (12 days) | 8,05 |
| 336 (14 days) | 7,92 |
| 384 (16 days) | 7,78 |
| 432 (18 days) | 7,77 |

Such as they are used in this document, the terms "about" and/or "approximately", when they refer to a value or characteristic, are meant to cover variations of ± 10 % of some embodiment, ± 5 % of some embodiment, relative to the specified value or characteristic, as said variations are appropriate to carry out the described composition and method/uses.

Although in this document the embodiments of the present invention have been described with reference to various specific features, it will be obvious for a skilled person to carry out the invention with modifications. All of these modifications are considered to be within the scope of the claims. Likewise, the claims are intended to cover all the generic and specific characteristics of the described exemplary embodiments and all the statements of the scope of protection which, by language reasons, may be said to be therewithin.

The scope of the present invention is set forth in the attached claims.

**Claims**

**1.** A composition comprising nanoparticles, wherein the nanoparticles are of polymeric material and include an antimicrobial agent comprising calcium hydroxide, **characterized in that**:

the polymeric material is selected from the group consisting of poly(L(+)-lactic-co-glycolic acid), PLGA; polylactic acid, PLA; polyglycolic acid, PGA; polycaprolactone, PCL; and/or combinations thereof;

the antimicrobial agent is encapsulated in the nanoparticles and the concentration of the antimicrobial agent is in the range of 0.1 to 5 mg/ml; and

the composition has a pH in the range of 8 - 13.

**2.** The composition according to claim 1, wherein the nanoparticles have a size lower than 300 nm, preferably of 150 nm.

**3.** The composition according to any one of the previous claims, wherein the polymeric material is PLGA, and the concentration of PLGA concentration is in a range from 5 to 20 mg/ml, preferably from 10 to 12 mg/ml.

**4.** The composition according to any one of the previous claims, wherein the nanoparticles have a polydispersion index lower than 0.2 and a negative surface charge.

**5.** The composition according to any one of the previous claims, wherein the polymeric material is biodegradable.

**6.** The composition according to any one of the previous claims, wherein the composition is a gel, an aqueous solution or is in the form of dispersed particles.

**7.** A method for the preparation of a composition comprising nanoparticles, the method comprising the following steps:

preparing an organic phase (101) that comprises adding a first mixture to a second mixture, wherein the first mixture comprises calcium hydroxide and a first solvent and wherein the second mixture comprises a polymeric material and a second solvent, wherein said polymeric material is selected from the group consisting of poly(L(+)-lactic-co-glycolic acid), PLGA; polylactic acid, PLA; polyglycolic acid, PGA; polycaprolactone, PCL; and/or combinations thereof;

preparing an aqueous phase (102) comprising a surfactant product and leaving the aqueous phase under magnetic stirring;

adjusting a pH of the aqueous phase (102) to a value above 10;

adding the organic phase (101) into the aqueous phase (102), drop by drop at a constant rate, wherein the mixture is left under magnetic stirring;

carrying out an evaporation process at low pressure (103) for removing said first and second solvent; and

obtaining the nanoparticles (104).

**8.** The method according to claim 7, further comprising lyophilizing the obtained nanoparticles.

**9.** The method according to any one of previous claims 7 or 8, wherein the first solvent comprises dimethyl sulfoxide, DMSO, and wherein the second solvent comprises acetone.

**10.** The method according to claim 9, wherein the second solvent comprises 4 times the volume of the first solvent.

**11.** The method according to any one of claims 7 to 10, wherein the polymeric material comprises PLGA, and wherein the calcium hydroxide quantity is double that of the organic phase (101) relative to the concentration of the nanoparticles (104) and the quantity of PLGA is double relative to the concentration of the nanoparticles (104).

**12.** The method according to any one of claims 7 to 11, wherein the pH value is adjusted to a value between 11 and 12.

**13.** The method according to any one of claims 7 to 12, wherein the drop rate of the organic phase (101) into the aqueous phase (102) is between 2 and 5 seconds, preferably 3 seconds.

**14.** The method according to any one of claims 7 to 13, wherein each drop has a volume of 50 microliters.

**15.** The composition according to any one of claims 1 to 6 for use in dental treatment, particularly: as an antibacterial compound for its placement inside the root canal, as a sealant of the root canal, for pulp capping, for repairing the dentin or dental-periodontal communication, as material for retrograding obturation in apical surgery and/or as indirect pulp capping and as cavitary base.


**Patentansprüche**

**1.** Zusammensetzung, die Nanopartikel umfasst, wobei die Nanopartikel aus polymerem Material bestehen und ein antimikrobielles Mittel einschließen, das Calciumhydroxid umfasst, **dadurch gekennzeichnet, dass**:

das polymere Material aus der Gruppe ausgewählt ist, die aus Poly(L(+)-lactid-co-glycolid), PLGA; Polymilchsäure, PLA; Polyglykolsäure, PGA; Polycaprolacton, PCL; und/oder Kombinationen davon besteht;
das antimikrobielle Mittel in den Nanopartikeln eingekapselt ist und die Konzentration des antimikrobiellen Mittels im Bereich von 0,1 bis 5 mg/mL liegt; und
die Zusammensetzung einen pH-Wert im Bereich von 8-13 aufweist.

**2.** Zusammensetzung nach Anspruch 1, wobei die Nanopartikel eine Größe von weniger als 300 nm, vorzugsweise von 150 nm, aufweisen.

**3.** Zusammensetzung nach einem der vorhergehenden Ansprüche, wobei das polymere Material PLGA ist und die PLGA-Konzentration in einem Bereich von 5 bis 20 mg/mL, vorzugsweise von 10 bis 12 mg/mL, liegt.

**4.** Zusammensetzung nach einem der vorhergehenden Ansprüche, wobei die Nanopartikel einen Polydispersionsindex von weniger als 0,2 und eine negative Oberflächenladung aufweisen.

**5.** Zusammensetzung nach einem der vorhergehenden Ansprüche, wobei das polymere Material biologisch abbaubar ist.

**6.** Zusammensetzung nach einem der vorhergehenden Ansprüche, wobei die Zusammensetzung ein Gel, eine wässrige Lösung oder in Form von dispergierten Partikeln ist.

**7.** Verfahren zur Herstellung einer Zusammensetzung, die Nanopartikel umfasst, wobei das Verfahren folgende Schritte umfasst:

Herstellen einer organischen Phase (101), die das Zugeben eines ersten Gemisches zu einem zweiten Gemisch umfasst, wobei das erste Gemisch Calciumhydroxid und ein erstes Lösungsmittel umfasst und wobei das zweite Gemisch ein polymeres Material und ein zweites Lösungsmittel umfasst, wobei das polymere Material aus der Gruppe ausgewählt ist, die aus Poly(L(+)-lactid-co-glycolid), PLGA; Polymilchsäure, PLA; Polyglykolsäure, PGA; Polycaprolacton, PCL; und/oder Kombinationen davon besteht;
Herstellen einer wässrigen Phase (102), die ein Tensidprodukt umfasst, und Belassen der wässrigen Phase unter magnetischem Rühren;
Anpassen eines pH-Wertes der wässrigen Phase (102) auf einen Wert über 10;
Zugeben der organischen Phase (101) in die wässrige Phase (102), wobei mit einer konstanten Rate zugetropft wird, wobei das Gemisch unter magnetischem Rühren belassen wird;
Durchführen eines Verdampfungsverfahrens bei niedrigem Druck (103) zum Entfernen des ersten und zweiten Lösungsmittels; und

Erhalten der Nanopartikel (104).

8. Verfahren nach Anspruch 7, das ferner das Gefriertrocknen der erhaltenen Nanopartikel umfasst.

9. Verfahren nach einem der vorhergehenden Ansprüche 7 oder 8, wobei das erste Lösungsmittel Dimethylsulfoxid, DMSO, umfasst und wobei das zweite Lösungsmittel Dimethylketon umfasst.

10. Verfahren nach Anspruch 9, wobei das zweite Lösungsmittel das 4-fache Volumen des ersten Lösungsmittels umfasst.

11. Verfahren nach einem der Ansprüche 7 bis 10, wobei das polymere Material PLGA umfasst, und wobei die Calciumhydroxidmenge doppelt so groß ist wie die der organischen Phase (101) in Bezug auf die Konzentration der Nanopartikel (104) und die PLGA-Menge doppelt so groß ist in Bezug auf die Konzentration der Nanopartikel (104).

12. Verfahren nach einem der Ansprüche 7 bis 11, wobei der pH-Wert auf einen Wert zwischen 11 und 12 angepasst wird.

13. Verfahren nach einem der Ansprüche 7 bis 12, wobei die Zutropfgeschwindigkeit der organischen Phase (101) in die wässrige Phase (102) zwischen 2 und 5 Sekunden, vorzugsweise 3 Sekunden, beträgt.

14. Verfahren nach einem der Ansprüche 7 bis 13, wobei jeder Tropfen ein Volumen von 50 Mikrolitern aufweist.

15. Zusammensetzung nach einem der Ansprüche 1 bis 6 zur Verwendung bei der zahnärztlichen Behandlung, insbesondere: als antibakterielle Verbindung zum Einbringen in den Wurzelkanal, als Versiegler des Wurzelkanals, zur Pulpaüberkappung, zur Reparatur der Dentin- oder Zahn-Parodontal-Verbindung, als Material zur retrograden Obturation in der apikalen Chirurgie und/oder zur indirekten Pulpaüberkappung und als Kavitätenbasis.

**Revendications**

1. Composition comprenant des nanoparticules, dans laquelle les nanoparticules sont en matériau polymère et comprennent un agent antimicrobien comprenant de l'hydroxyde de calcium, **caractérisée en ce que** :

le matériau polymère est choisi parmi le groupe constitué du poly(acide L(+)-lactique-co-glycolique), PLGA ; du poly(acide lactique), PLA ; du poly(acide glycolique), PGA ; de la polycaprolactone, PCL ; et/ou des combinaisons de ceux-ci ;
l'agent antimicrobien est encapsulé dans les nanoparticules et la concentration de l'agent antimicrobien est comprise entre 0,1 et 5 mg/ml ; et
la composition a un pH compris entre 8 et 13.

2. Composition selon la revendication 1, dans laquelle les nanoparticules ont une taille inférieure à 300 nm, de préférence de 150 nm.

3. Composition selon l'une quelconque des revendications précédentes, dans laquelle le matériau polymère est le PLGA, et la concentration de PLGA est comprise entre 5 et 20 mg/ml, de préférence entre 10 et 12 mg/ml.

4. Composition selon l'une quelconque des revendications précédentes, dans laquelle les nanoparticules ont un indice de polydispersion inférieur à 0,2 et une charge de surface négative.

5. Composition selon l'une quelconque des revendications précédentes, dans laquelle le matériau polymère est biodégradable.

6. Composition selon l'une quelconque des revendications précédentes, dans laquelle la composition est un gel, une solution aqueuse ou se trouve sous forme de particules dispersées.

7. Procédé pour la préparation d'une composition comprenant des nanoparticules, le procédé comprenant les étapes suivantes :

préparation d'une phase organique (101) comprenant l'ajout d'un premier mélange à un deuxième mélange, dans lequel le premier mélange comprend de l'hydroxyde de calcium et un premier solvant et dans lequel le deuxième mélange comprend un matériau polymère et un deuxième solvant, dans lequel ledit matériau polymère est choisi parmi le groupe constitué de poly(acide L(+)-lactique-co-glycolique), PLGA ; du poly(acide lactique), PLA ; du poly(acide glycolique), PGA ; de la polycaprolactone, PCL ; et/ou des combinaisons de ceux-ci ;

préparation d'une phase aqueuse (102) comprenant un produit tensioactif et laisser la phase aqueuse sous agitation magnétique ;

ajustement du pH de la phase aqueuse (102) à une valeur supérieure à 10 ;

ajout de la phase organique (101) dans la phase aqueuse (102), goutte à goutte, à un rythme constant, dans lequel le mélange est soumis à une agitation magnétique ;

mise en oeuvre d'un processus d'évaporation à basse pression (103) pour éliminer lesdits premier et deuxième solvants ; et

obtention des nanoparticules (104).

8. Procédé selon la revendication 7, comprenant en outre la lyophilisation des nanoparticules obtenues.

9. Procédé selon l'une quelconque des revendications précédentes 7 ou 8, dans lequel le premier solvant comprend du diméthylsulfoxyde, DMSO, et dans lequel le deuxième solvant comprend de l'acétone.

10. Procédé selon la revendication 9, dans lequel le deuxième solvant comprend 4 fois le volume du premier solvant.

11. Procédé selon l'une quelconque des revendications 7 à 10, dans lequel le matériau polymère comprend du PLGA, et dans lequel la quantité d'hydroxyde de calcium est double de celle de la phase organique (101) par rapport à la concentration des nanoparticules (104) et la quantité de PLGA est double par rapport à la concentration des nanoparticules (104).

12. Procédé selon l'une quelconque des revendications 7 à 11, dans lequel la valeur du pH est ajustée à une valeur comprise entre 11 et 12.

13. Procédé selon l'une quelconque des revendications 7 à 12, dans lequel la vitesse de chute de la phase organique (101) dans la phase aqueuse (102) est comprise entre 2 et 5 secondes, de préférence 3 secondes.

14. Procédé selon l'une quelconque des revendications 7 à 13, dans lequel chaque goutte a un volume de 50 microlitres.

15. Composition selon l'une quelconque des revendications 1 à 6 pour son utilisation dans le traitement dentaire, en particulier : comme composé antibactérien pour son placement à l'intérieur du canal radiculaire, comme scellant du canal radiculaire, pour le coiffage de la pulpe, pour la réparation de la dentine ou de la communication dento-périodontale, comme matériau pour l'obturation rétrograde dans la chirurgie apicale et/ou comme coiffage indirect de la pulpe et comme base cavitaire.

101

102

Low-pressure
evaporation

103

104

**Fig. 1**

Centrifugation

**Fig. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8105086 B
- US 2017135960 A

- KR 101879399

**Non-patent literature cited in the description**

- **B.I. CERDA-CRISTERNA et al.** PLGA-based nanoparticles for sustained release of Ca++ for apexification. *Abstracts of the Academy of Dental Materials Annual Meeting, 12-15 October 2016 - Chicago, USA,* 12 October 2016

- **CERDA-CRISTERNA et al.** Sustained release of calcium hydroxide from poly(DL-lactide-co-glycolide) acid microspheres for apexification. *Odontology,* 10 September 2016, vol. 4 (3), 318-23
- **XIAOMAN et al.** Chitosan-decorated calcium hydroxide microcapsules with pH-triggered release for endodontic applications. *J Mater Chem B.,* 07 December 2015, vol. 3 (45), 8884-8891